(19) Europäisches Patentamt
European Patent Office
Office européen des brevets

(11) **EP 3 901 964 B1**

(12) **EUROPEAN PATENT SPECIFICATION**

(45) Date of publication and mention
of the grant of the patent:
**06.11.2024 Bulletin 2024/45**

(51) International Patent Classification (IPC):
***G16H 40/63*** *(2018.01)* ***G16H 30/20*** *(2018.01)*

(21) Application number: **21165484.3**

(22) Date of filing: **29.03.2021**

(52) Cooperative Patent Classification (CPC):
**G16H 30/20; G16H 40/63**

(54) **INTELLIGENT SCAN RECOMMENDATION FOR MAGNETIC RESONANCE IMAGING**

INTELLIGENTE SCAN-EMPFEHLUNG FÜR DIE MAGNETRESONANZTOMOGRAPHIE

RECOMMANDATION INTELLIGENTE DE SCAN POUR L'IMAGERIE PAR RÉSONANCE MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2020 US 202063013825 P**

(43) Date of publication of application:
**27.10.2021 Bulletin 2021/43**

(73) Proprietor: **Siemens Healthineers AG
91301 Forchheim (DE)**

(72) Inventors:
• **Arroyo Camejo, Silvia Bettina
90763 Fürth (DE)**
• **Forman, Christoph
91052 Erlangen (DE)**
• **Kober, Tobias
1007 Lausanne (CH)**

(74) Representative: **Siemens Healthineers
Patent Attorneys
Postfach 22 16 34
80506 München (DE)**

(56) References cited:
**US-A1- 2019 320 934**

• **LEE YOUNG HAN: "Efficiency Improvement in a Busy Radiology Practice: Determination of Musculoskeletal Magnetic Resonance Imaging Protocol Using Deep-Learning Convolutional Neural Networks", JOURNAL OF DIGITAL IMAGING, SPRINGER-VERLAG, CHAM, vol. 31, no. 5, 4 April 2018 (2018-04-04), pages 604 - 610, XP036596714, ISSN: 0897-1889, [retrieved on 20180404], DOI: 10.1007/S10278-018-0066-Y**

## Description

REFERENCE TO PROVISIONAL APPLICATION

This application claims priority to Provisional Application US 63/013,825 filed on April 22, 2020.

## TECHNICAL FIELD

The present disclosure relates to techniques for developing a workflow for magnetic resonance imaging (MRI), and in particular to techniques for recommending MR protocols (MR is an abbreviation of magnetic resonance using various data sources, which may be part of an automated process.

## BACKGROUND

Magnetic resonance imaging (MRI) is a medical imaging technique that provides diagnostically relevant information of biological tissue. Its diagnostic relevance originates from the informative power and versatility of this imaging modality, which offers differentiated and precise structure information in a non-invasive fashion. Magnetic resonance imaging is based on the controlled manipulation of nuclear spins inside a patient's body and subsequent detection of the nuclear spin response. The space-dependent encoding of the spin response allows for a reconstruction of the patient's structural composition and functional constitution for the radiologist to perform diagnostic reading.

Conventionally, to generate MR images for diagnosis, the MRI technician conducting the MR scan selects a sequential arrangement of MR protocols from an MR scanner library of imaging protocols, which may be based, e.g., on a relevant body region, a suspected disease (e.g., through the clinical indication of the referring physician or the radiologist), patient-specific requirements (e.g., implants, motion-related incompliance), and preferences of the clinical institution and radiologists who will later perform reading of the MR images for diagnosis. In addition, during the scan procedure the technician typically performs checks to ensure that the image quality of the recorded scans allows for diagnosis. The technician may then make protocol modifications, e.g. adapt the field-of-view to the patient's body and others and will perform re-scans, if required.

This classical MRI scan workflow requires MRI technicians to undergo intense modality-specific training and acquire many years of scanning experience. Today, the number of performed MR scans is continuously increasing while qualified personnel is scarce, which contributes to high operational costs. And even for highly trained technicians, the high number of manual steps required to optimally perform MR scanning together with increasing time pressure causes significant risks for operational errors. Even in high profile hospitals, there might be times when less trained technicians have to operate the scanner, for example at night or at the weekend.

In US 9,928,589 B2, an apparatus and a method for supporting acquisition of a multi-parametric image is described. The apparatus comprises a disease selector configured to select a suspected disease of a patient based on patient information; and an image selector configured to determine a set of imaging conditions of a multi-parametric magnetic resonance image corresponding to the suspected disease based on a multi-parametric magnetic resonance imaging model. However, often, it is very hard to determine the disease of a patient before having recorded medical images.

In US 2018/ 0 101 644 A1, a method for the provision of confidence information about a diagnosis of a diagnosis system based on medical image data is described. The diagnosis system is designed as an algorithm that uses as its input the medical image data and further patient specific data. The measure of confidence is determined by execution of a determination algorithm that has as its input parameter a characteristic value for the diagnosis system, for example a sensitivity or a specificity of the diagnosis system, and/or an image content from medical image data. Also a further acquisition of additional image data can be proposed based on the confidence information. The additional medical image data can be acquired such that the data exhibit a changed image contrast with respect to the already existing medical image data.

In US 2019/ 320 934 A1, a medical image acquisition with sequence prediction using deep learning is described. First, an initial scout sequence is performed of a patient. The initial scout sequence is validated and an abbreviated acquisition protocol is performed. The abbreviated acquisition protocol is validated and additional sequences are performed. Subsequent sequences are configured based on the analysis of the previous scans using deep learning-based reasoning to select the next appropriate settings and procedures. In an embodiment, the identification of abnormal regions in the initial scout sequence is used to determine one or more additional acquisition sequences.

In LEE YOUNG HAN: "Efficiency Improvement in a Busy Radiology Practice: Determination of Musculoskeletal Magnetic Resonance Imaging Protocol Using Deep-Learning Convolutional Neural Networks", JOURNAL OF DIGITAL IMAGING, SPRINGER-VERLAG, CHAM, vol. 31, no. 5, 4 April 2018 (2018-04-04), pages 604-610, XP036596714, ISSN: 0897-1889, DOI: 10.1007/S10278-018-0066-Y) a determination of musculoskeletal magnetic resonance imaging protocol using deep-learning convolutional neural networks is described. In detail, a classifier based on short-text classification is used for determining imaging protocols. The text classification is applied to tumor/infection and routing protocols.

## SUMMARY

Again, as of today, most MR scans are performed manually, requiring the technician to select the required protocols from a protocol library. In an attempt to automate the MR imaging workflow, applications have been designed containing a sequential arrangement of protocols specifically composed for the imaging of a specific anatomical region and/or suspected disease. In particular, the steps of image slice planning can already be automated by means of pattern matching algorithms. However, as of today, there is no software that can enable an untrained technician to perform an entire MR scan with a high success rate while considering patient-specific needs or findings.

Hence, there is an object of the invention to find a solution for a preparation and execution of an MR scan without the need of a qualified operating person.

The above-mentioned problem is solved by a scan recommendation method according to claim 1, a personal scan preparation method according to claim 5, a medical imaging method according to claim 8, a scan workflow performing method according to claim 9, a scan recommendation system according to claim 10, a personal scan preparation system according to claim 14, a medical imaging system according to claim 17 and a scan workflow performing system according to claim 18.

The scan recommendation method according to the invention comprises the step of receiving evaluation data comprising classification data. The classification data classify anomalies of image derived metrics, which were detected based on input data concerning the medical condition of a patient. Preferably, the anomalies are detected in medical image data of the patient. A classification comprises a type of identification of a detected anomaly, assigning probability values for different possible types of anomalies to the detected anomaly. For example, the anomalies can be anomalies detected in the input data, for example medical image data, or they can comprise information about anomalies based on medical data about the patient of a different type. Anomalies detected in image data comprise detected image abnormalities or distinctive image features such as hyperintense areas, hypointense areas, abnormal tissues, midline shift, vascular stenosis, plaques, demyelination or mass effects. As explained further below in the text, anomalies may also be detected image metric abnormalities or distinctive features. Then the next recommended MR protocol, which is intended to be executed with an MR scan of the patient, is automatically determined based on the evaluation data. As explained below, the evaluation is not limited to the evaluation of image data. Hence, a plurality of different type of input data about a patient's condition can be analysed to determine the evaluation data. Also, a plurality of data sets of a single special type of input data concerning a patient's condition can be evaluated. The scan recommendation can be based on receiving a plurality of evaluation data of different types. The detection of the anomalies can be realized based on machine learning methods, for example based on neural networks such as a convolutional neural network taking the input data, for example image data, as input and providing as output the probability of the input data to be normal versus anomalous. Also the classification of the detected anomalies can be realized using machine learning methods, for example neural network-based techniques, or more specifically using a convolutional neural network. If the probability value of a detected anomaly for the received input data, for example image data, is higher than a certain customizable threshold value $\xi_d$, then the input data, for example image data, assigned to the detected anomaly are taken as input for the subsequent classification step, as mentioned above. The scan recommendation can also be realized based on machine learning based techniques, especially neural network techniques as below explained. In the simplest variant, the evaluation data simply include the classification data. However, the evaluation data may additionally comprise also different types of data. The method according to the invention allows the trained or untrained technician to perform a highly personalized MRI scan workflow on each patient, generating an optimal set of MR images that are required for diagnosis of that patient's condition. The method may provide a basis for a fully autonomously optimized MR imaging workflow yet allowing for manual or electronic customization thereof as desired by the operator. The MR scanner operator may choose to run the MR scan workflow either in fully autonomous, fully manual, or in a hybrid mode.

The personal scan preparation method according to the invention comprises the steps of receiving input data, for example medical image data, concerning the medical condition of a patient, wherein the input data comprise image-derived metrics based on image data. Evaluation data based on the input data are determined by detecting anomalies of image derived metrics in the input data, determining classification data based on the detected anomalies as evaluation data and performing the scan recommendation method according to the invention. Advantageously, the classification of the anomalies can be used to adapt an MR protocol to a special kind of anomaly. For example, a special contrast is chosen for the recommended MR protocol. Further, the personal scan preparation method shares the advantages of the scan recommendation method.

The medical imaging method according to the invention comprises the steps of determining a next recommended protocol based on the scan recommendation method according to the invention and transmitting the next recommended protocol to an MR scan system and performing an MR scan by the MR scan system based on the received protocol. The medical imaging method shares the advantages of the scan recommendation method according to the invention.

The scan workflow performing method according to the invention comprises the steps of performing the medical imaging method according to the invention. After the MR scan of the medical imaging method, anomalies of image derived metrics are detected in the reconstructed MR-image data and classification data are determined based on the detected anomalies. Then the aforementioned three steps are iteratively carried out taking into account the determined classification data, until an abortion criterion is attained. In other words, first a next recommended protocol is determined based on the scan recommendation method of the invention or the personal scan preparation method according to the invention. Then, the next recommended protocol is transmitted to an MR scan system and the MR scan is performed by the MR scan system based on the recommended protocol. Further, an iterative protocol determination and measurement is performed, comprising the steps of controlling the MR scan, evaluating the reconstructed MR-image data and iteratively improving input data for the next recommended protocol based on classification of anomalies of the reconstructed MR-image data. For example, the operator can first record a standard set of MR protocol and employ the scan recommendation method based on the recorded first images for additional MR scans that might be additionally indicated for the current patient based on the imaging information. Advantageously a complete automatic workflow for an optimized MR scan of a patient is realized, wherein the protocol for the MR imaging and hence the MR imaging itself is optimized with each iteration loop. It has to be mentioned that all the methods described above, i.e. the scan recommendation method, the personal scan preparation method, the medical imaging method and the scan workflow performing method can also be carried out in the case that the operator does not sit in front of the scanner console. Hence, all of the described methods can be performed without even a console room next to the MR scan system, i.e. fully automated, or the monitoring operator is sitting somewhere else and controls remotely. Advantageously, a skilled operator can perform an investigation of a patient and a surveillance of a medical imaging process remotely and therefore professional skills can be accessed where an MR system is available.

The scan recommendation system according to the invention comprises at least one interface, preferably a plurality of input interfaces, for receiving evaluation data. The evaluation data comprise classification data, wherein the classification data classify anomalies of image derived metrics detected based on input data, wherein the input data concern the medical condition of a patient. Further, the scan recommendation system according to the invention includes a recommendation unit for automatic determination of a next recommended MR protocol to be executed in accordance with an MR scan for determining a next recommended MR protocol to be executed in accordance with an MR scan of the patient based on the received evaluation data.

In other words, the scan recommendation system can be configured to fully and automatically recommend the next, diagnostically most insightful MR protocol for measurement. The evaluation data for the recommendation system may be based on either a full set or subsets of inputs including image data (historical or current), image derived metrics, for example geometric properties of tissue and cavities like vascular wall thickness, cross section, and ventricular volume etc., electronic health record information, and/or user interface input. The scan recommendation system according to the invention shares the advantages of the scan recommendation method according to the invention.

The personal scan preparation system according to the invention comprises an input interface for receiving input data concerning the medical condition of a patient, wherein the input data comprise image-derived metrics based on image data. Further, the personal scan preparation system includes an evaluation unit, which comprises a detection unit for detecting anomalies in the image-derived metrics in the input data, a classification unit for determining classification data based on the detected anomalies as evaluation data, and the scan recommendation system according to the invention. The personal scan preparation system shares the advantages of the personal scan preparation method according to the invention.

The medical imaging system according to the invention comprises an MR scan system and a scan recommendation system or a personal scan preparation system according to the invention, which is coupled to the MR scan system to determine a next recommended protocol and to send the next recommended protocol to the MR scan system for performing an MR scan. The medical imaging system according to the invention shares the advantages of the medical imaging method according to the invention.

The scan workflow performing system comprises the medical imaging system according to the invention. Further, the scan workflow performing system includes an anomaly detection unit for detecting anomalies of image-derived metrics in the reconstructed MR-image data. Furthermore, the scan workflow performing system comprises a classification unit for determining classification data based on the detected anomalies. The scan workflow performing system also comprises an iteration unit for determining, based on the determined classification data, if iteratively performing the three steps of medical imaging, anomaly detection and anomaly classification, until an abortion criterion is attained. The scan workflow performing system shares the advantages of the scan workflow performing method according to the invention.

The essential components of the scan recommendation system according to the invention, the personal scan preparation system according to the invention, the medical imaging system according to the invention and the scan workflow performing system according to the invention can for the most part be designed in the form of software components. This applies in particular to the recommendation unit of the scan recommendation system, which can comprise for example a fully connected neural network or a random forest, the evaluation unit, the detection unit and the classification unit of the personal scan preparation system and the anomaly detection unit and the iteration unit of the scan workflow performing system, but also parts of the input interfaces. In principle, however, some of these components can also be implemented in the form of software-supported hardware, for example FPGAs or the like, especially when it comes to particularly fast calculations. Likewise, the required interfaces, for example if it is only a matter of transferring data from other software components, can be designed as software interfaces. However, they can also be designed as hardware-based interfaces that are controlled by a suitable software. Furthermore, some parts of the above-mentioned components may be distributed and stored in a local or regional or global electronic network or a combination of a network and software, in particular a cloud system.

A largely software-based implementation has the advantage that medical imaging systems including a magnetic resonance imaging system that have already been used can easily be retrofitted by a software update in order to work in the manner according to the invention. In this respect, the object is also achieved by a corresponding computer program product with a computer program that can be loaded directly into a memory device, for example a control device of a medical imaging system, with program sections, in order to carry out all steps of the method according to the invention, if the program is executed in the medical imaging system, in particular the control device. In addition to the computer program, such a computer program product may contain additional components such as a documentation and/ or additional components, including hardware components such as Hardware keys (dongles etc.) for using the software.

For transport to the medical imaging system and/or for storage on or in the medical imaging system, a computer-readable medium, for example a memory stick, a hard disk or some other transportable or permanently installed data carrier is used on which the program sections of the computer program that can be read in and executed by a computer unit of the medical imaging system are stored. The computer unit can comprise for example, one or more cooperating microprocessors or the like used for this purpose. The computer program can also be acquired by download from a central webshop of the applicant or another internet based source or a cloud based source.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the scan recommendation method according to the invention the automatic scan recommendation is based on using at least one of the following software types or types of a combination of software and hardware:

- a machine learning model, preferably including at least one of:

  - a shallow neural network model,
  - a deep neural network model,
  - a convolutional neural network,
  - a fully connected neural network,
  - a support vector machine,
  - a decision tree,
  - a random forest,

- an expert system,
- a classical model algorithm.

For example, the scan recommendation method may be carried out using a trained model (for example a fully connected neural network or a random forest). The trained model receives the above-mentioned evaluation data, which for example comprise classified anomalies. Hence, the trained model is used to determine a next recommended MR protocol to be executed in accordance with an MR scan of a patient. The mentioned input data, for example image data of the patient, can be information of all kind about the patient, which may influence an imaging process to be planned. The mentioned input data can either be directly used as input for the trained model or can undergo one or several preprocessing step, for example by a machine learning or deep learning model, before they are fed into the trained model.

Preferably, in the scan recommendation method according to the invention, the evaluation data comprise a first possible scan workflow direction determined by mapping topics, based on extracted keywords in the input data concerning a medical condition of the patient, onto probabilities of a next recommended protocol. The input data can be for example written information, i.e. text data about a suspected diagnosis, symptoms, an indication referring to the symptoms or a body region, where the symptoms are localized and to which the indication or suspected diagnosis refers. The input data can comprise electronic health record information comprising at least one of a suspected diagnosis, a suspected disease, relevant observations of the technician, an indication from referring clinician, a body region, patient data, or an MR protocol. Hence, electronic health record information can add additional information about the health condition of a patient and can be used for modifying an MR imaging protocol. Further, the protocol can be modified by direct input by the user. Therefore, the input data can also include data input by the user. The keyword extraction can be performed based on an expert system or a machine learning approach or a deep learning approach or combinations thereof. The extracted keywords can be determined based on data preprocessing, for example lemmatization or stemming and bag-of words generation. The topics are determined by a topic detection algorithm or a generated topic model. The topic detection can be realized by a trained unsupervised topic detection with latent Dirichlet allocation. Latent Dirichlet allocation can be carried out using classical machine learning methods, for example a hierarchical Bayesian model trained with variational inference as decribed in D. Blei et al. JMLR 3, 2003, or using neural variational inference as decribed in Y. Miao et al. Proc. ICML, 2017. The mapping of the topics onto a possible scan workflow direction, i.e. a set of possible recommended next scan protocols can be based on expert knowledge, customer preference or an additional designated machine learning algorithm or deep learning algorithm. To each of the topics a set of recommended next scan protocols with values of probabilities is assigned.

Further, also a second possible scan workflow direction can be determined by the scan recommendation method according to the invention, wherein the classification data, which are included in the evaluation data, are mapped onto probability values of a next recommended protocol. Such a mapping can be realized using a mapping model, for example a random forest or a fully connected neural network. Then, the final recommendation step is performed based on the second possible scan workflow direction and optionally additionally based on the first possible scan workflow direction. The final recommending step can be realized using a weighted averaging of the first and the second possible scan workflow direction or using a fully connected neural network.

In a variant of the personal scan preparation method according to the invention, the input data comprise image data and the step of detecting anomalies comprises detecting anomalies in the image data. As mentioned above, the detection and classification of anomalies in image data can be realized using a neural network, for example a convolutional neural network. The anomalies can be direct anomalies of image data or anomalies of metrics related to image data. Therefore, the input data comprise image derived metrics based on image data and the step of detecting anomalies comprises detecting anomalies in the image-derived metrics. Image derived metrics include geometric properties of tissues and cavities, potentially compared to normative data from a cohort. Such derived metrics encompass vascular wall thickness, cross sections of blood vessels and ventricular volume, grey mass volume in the brain and white mass volume in the brain.

As well preferred, in the personal scan preparation method, the input data can also comprise electronic health record information and/or user interface input data and the step of determining evaluation data can comprise the steps of extracting keywords from the electronic health record information and/or the user interface input, determining topics based on the extracted keywords and determining the first possible scan workflow direction by mapping the topics onto probabilities of a next recommended protocol. Keywords can comprise for example information regarding at least one of a suspected diagnosis, a suspected disease, relevant observations of the technician, an indication from referring clinician, a body region, patient data, or an MR protocol. The keyword extraction can be performed based on an expert system or a machine learning approach or a deep learning approach or combinations thereof. The scan recommendation can be realized by one or several classical and or machine learning algorithms. As mentioned above, electronic health record information can add additional information about the health condition of a patient and can be used for modifying an MR imaging protocol. Further, the MR imaging protocol can be modified by direct input by the user. Using a keyword extraction enables an automatic analysis of electronic health record information and/or the user interface input concerning MR protocol related content. Advantageously, also information concerning former medical examination can be integrated into the scan recommendation based on written text or direct protocol data input by a user. In this way, the data base for the automatic determination of a next recommended MR protocol is being broadened.

As mentioned above, the different types of information can be combined as a base for automatic determination of a next recommended protocol. The aforementioned listed input data may include patient information, user information and hospital information, which may either be directly used as input to the scan recommendation system or may undergo one or several preprocessing steps (e.g., by a machine learning or deep learning model) before being fed into the scan recommendation system.

In a variant of the scan recommendation system according to the invention, the recommendation unit comprises at least one of:

- a machine learning model, including at least one of:

    - a shallow neural network model,
    - a deep neural network model,
    - a convolutional neural network,
    - a fully connected neural network,
    - a decision tree,
    - a random forest,
    - a support vector machine,

- an expert system,
- a classical model algorithm.

The described variant of a scan recommendation system shares the advantages of the corresponding variant of a scan recommendation method.

The scan recommendation system according to the invention can also comprise an input interface for receiving evaluation data comprising a first possible scan workflow direction determined by mapping topics based on extracted keywords concerning a medical condition of the patient, onto probability values of a next recommended protocol. The described variant of a scan recommendation system shares the advantages of the corresponding variant of a scan recommendation method.

In an alternative variant of the scan recommendation system according to the invention, the recommendation unit comprises a mapping unit for determining a second possible scan workflow direction by automated mapping of the classification data onto probability values of a next recommended protocol and a final protocol recommendation unit for final recommending the next recommended protocol based on the second possible scan workflow direction and optionally additionally based on the first possible scan workflow direction. The described variant of a scan recommendation system shares the advantages of the corresponding variant of a scan recommendation method.

In a variant of the personal scan preparation system according to the invention, the input interface is configured to receive input data comprising the medical image data and the detection unit is configured to detect anomalies in the image data. The input interface can also be configured to receive input data comprising image derived metrics based on image data and the detection unit can be configured to detect anomalies in the image-derived metrics. The variants of the personal scan preparation system share the advantages of the corresponding variants of a personal scan preparation method.

In a further variant of the personal scan preparation system according to the invention, the input interface is configured to receive input data comprising electronic health record information and/or user interface input data. In that variant, the evaluation unit comprises an extraction unit for extracting keywords from the electronic health record information and/or the user interface input, a topic finding unit for determining topics based on the extracted keywords and a mapping unit for determining the first possible scan workflow direction by mapping the topics onto probability values of a next recommended protocol as evaluation data. The described variant of the personal scan preparation system shares the advantages of the corresponding variant of the personal scan preparation method according to the invention.

## BRIEF DESCRIPTION OF THE DRAWINGS/FIGURES

The above and other features and advantages of the present disclosure will be more apparent to those of ordinary skill in the art from the detailed description of preferred embodiments of the present disclosure with reference to the accompanying drawings, in which:

Figure 1 illustrates a flow chart of a scan preparation method according to an embodiment of the invention,

Figure 2 illustrates a flow chart of particular steps of the method illustrated in Figure 1,

Figure 3 illustrates a flow chart of a scan workflow performing method according to an embodiment of the invention,

Figure 4 illustrates a block diagram showing a personal scan preparation system with a scan recommendation system according to an embodiment of the invention,

Figure 5 illustrates a block diagram showing a medical imaging system with the personal scan preparation system as depicted in Figure 4,

Figure 6 illustrates a block diagram showing a scan workflow performing system according to the invention,

Figure 7 illustrates an example for an image data anomaly classification in detail,

Figure 8 illustrates an example for a metrics anomaly classification in detail,

Figure 9 illustrates an example for a keyword analysis based on electronic health record input data or user interface input data in detail,

Figure 10 illustrates the function of a scan recommendation according to an embodiment of the invention in detail.

## DETAILED DESCRIPTION

In order to make the object, technical solutions, and advantages of the present disclosure more apparent, the present disclosure will be further described in detail by way of embodiments hereinafter.

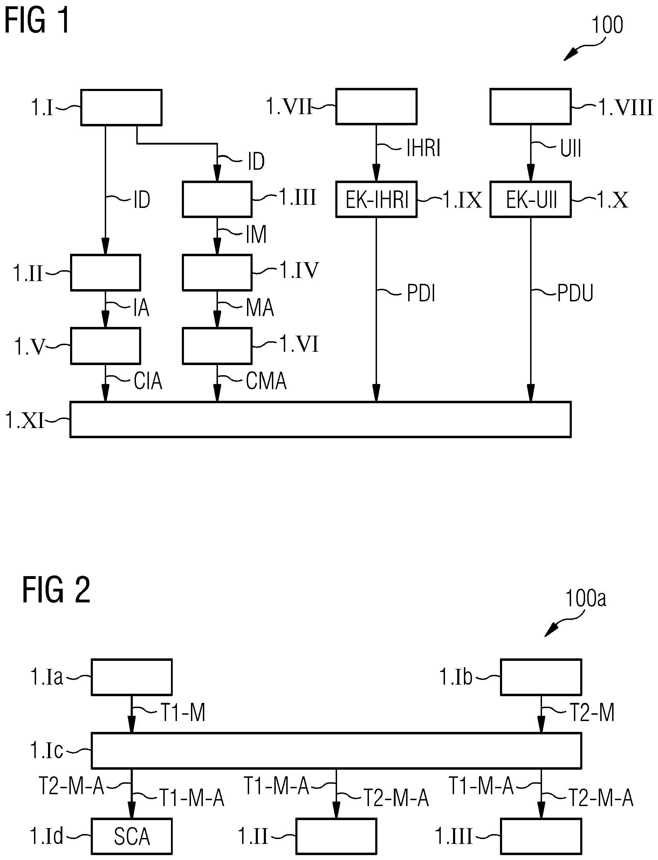

Figure 1 illustrates a flow chart 100 of a scan preparation method according to an embodiment of the invention for recommending a workflow for medical imaging of a patient. As shown in Figure 1, in step 1.I of the scan preparation method, image data ID from the patient to be examined are acquired. The image data ID may be acquired by an initial scan for creating a T1-map and a T2-map. Further, the image data ID may be received from historical images acquired from the patient in former times. In step 1.II, the image data ID are directly used as first input data for determination of evaluation data. The image data are analysed regarding image anomalies IA. The detection of anomalies IA in the image data ID can be realized using a convolutional neural network. In step 1.III, the image data ID are first subjected to pre-diagnostic analysis steps, wherein some metrics IM of the image data ID are derived. For example some pre-diagnostic analysis data such as, for instance, geometric properties of tissue and cavities like vascular wall thickness, cross section, and ventricular volume etc. are detected and measured and are compared to normative data from a cohort. Further, in step 1.IV, these image metrics IM are used as second input data and are subjected to an anomaly detection using a fully connected neural network, wherein some anomalies MA of metrics IM can be detected. In step 1.V, the image anomalies IA are further classified using the above-mentioned convolutional neural network. In step 1.VI, the detected anomalies MA of metrics IM are classified using for example a fully connected neural network, a decision tree or a random forest. Hence, in step 1.V and step 1.VI, evaluation data comprising classification data CIA, CMA for image anomalies IA and metrics anomalies MA are provided for later step 1.XI, which is the actual scan recommendation step. The classification data CIA concerning image anomalies IA comprise sets of probability values P(i) for the probability of the existence of different image anomalies IA in the image data ID input to the scan preparation method in step 1.I. The classification data CMA concerning metrics anomalies MA comprise sets of probability values P(j) for the probability of the existence of different metrics anomalies IM, which can also be understand as input data provided to the scan preparation method in step 1.IV.

In other words, the initial image data ID are recorded to provide a first measurement step benchmark and subsequently input these image data ID for an anomaly detection and anomaly classification. Quality assurance steps following each measurement can be employed to ensure sufficient image quality and trigger rescans, if required (e.g., due to patient motion). The anomaly detection and anomaly classification may be implemented, e.g., by a convolutional neural net, possibly as independent implementations. If anomaly detection/classification is applied, embodiments include advantageously using quantitative MR imaging protocols, for example for T1-maps and T2-maps for the measurement of the initial input images to provide a standardized image input for the anomaly detection and classification.

In step 1.VII, some electronic health record information IHRI is used as third input data, which comprises data from various sources such as objects, for example suspected diagnoses, referring indications, etc., and/or other data sources such as RIS, patient data, etc. In step 1.VIII, some additional information UII, also named as user interface input UII that comprises data indicating, for example, suspected diagnoses, referring indications, a body region(s), a desired MR protocol, etc., are acquired as fourth input data for the personal scan preparation method. In step 1.IX, the input electronic health record information IHRI is processed using, for example, natural language processing (NLP) to perform keyword extraction. By this way, extracted keywords EK-IHRI of input electronic health record information IHRI are acquired. Further continuing the personal scan preparation method, the user interface input UII is processed using, for example, NLP to additionally or alternatively extract keywords EK-UII in step 1.X. As above-mentioned and later described in more detail, the extracted keywords EK-IHRI, EK-UII are then assigned to different topics and the topics are then mapped to sets of probability values P(n), P(o), which represent a possible scan workflow direction PDI, PDU. Then the possible scan workflow directions PDI, PDU are provided as third and fourth evaluation data to the automatic scan recommendation method of step 1.XI. In step 1.XI, an automatic scan recommendation according to an embodiment of the invention is performed based on a combination of various evaluation values CIA, CMA, PDI, PDU to identify the next recommended protocol, as further discussed herein. The automatic scan recommendation can be realized based on a classical or machine learning system, such as a fully connected neural network (FCNN). However, the scan recommendation is not limited to this implementation and may be implemented in accordance with any suitable type of classical algorithm, expert system, or machine learning design that may process inputs, e.g. in accordance with classical, training, or learning processes to recommend protocols based upon specific type of inputs.

In Figure 2, a flow chart 100a, illustrating step 1.I as shown in Figure 1, is depicted. In step 1.Ia, a T1-map measurement is carried out based on an initial MR scan of the patient and a T1-map T1-M is acquired. The initial scan of the patient can be done with low resolution and does not take much time compared to the main imaging process of the examination. Further, in step 1.Ib a T2-map measurement is carried out based on the initial MR scan of the patient and a T2-map T2-M is acquired. In step 1.Ic, a quality analysis of the acquired data T1-M, T2-M is performed and some quality proved image data T1-M-A, T2-M-A are provided to further process and analysis. In Step 1.Id the acquired data T1-M-A, T2-M-A, which are comprised by the image data ID mentioned in context with step 1.I, are used for generation of synthetic contrast images SCA. Further, the acquired data T1-M-A, T2-M-A are also used as image data ID in steps 1.II and 1.III as above-discussed in detail in context with Figure 1.

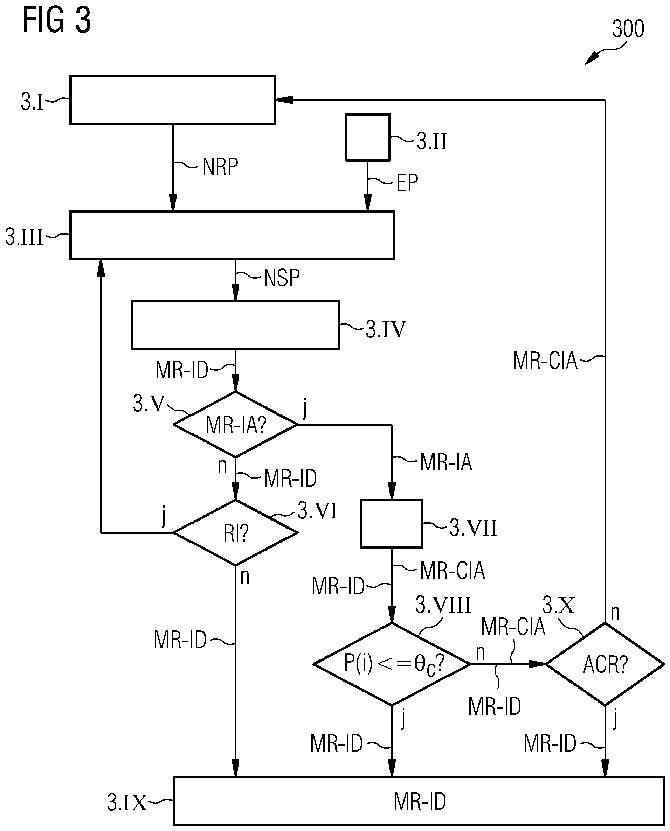

In Figure 3, a flow chart 300 is depicted, which illustrates a scan workflow performing method comprising the above-mentioned personal scan preparation method including a scan recommendation of a workflow for a scan and an additional iterative protocol determination and measurement process that may run until a relevant scan abortion criterion combination is fulfilled. The flow chart 300 begins with step 3.I, which includes the complete personal scan preparation method as explained in detail in context with Figure 1 comprising step 1.I to step 1.XI. Hence, in step 3.I the method as described in Figure 1 is performed and a next recommended protocol NRP is created based on the input data ID, IM, IHRI, UII, which are analysed and processed as described in context with Figure 1. The next recommended protocol NRP is put into a scan queue. Further, in step 3.II, some additional manually or electronically selected protocols EP to be scanned are added to the scan queue due to for example clinic standard, health insurance guidelines, radiologist or technician preferences. Then in step 3.III, the next scan protocol NSP is transmitted from the scan queue to an MR scan unit. In step 3.IV the actual scan process for the examination of the patient and acquisition of raw data as a basis for the reconstruction of image data MR-ID with high resolution and quality from a patient is performed. In step 3.V, the reconstructed image data MR-ID are then analysed concerning potential anomalies MR-IA in the image data MR-ID. In case some anomalies MR-IA are detected, which is symbolized in Figure 3 with "j", the process continues with step 3.VII. In case no anomalies MR-IA are detected, which is symbolized in Figure 3 with "n", the process continues with step 3.VI. In step 3.VI, it is determined, if any additional item RI remains in the queue, which means that a protocol remains in the queue, which has to be worked off. If this is the case, which is symbolized in Figure 3 with "j", the process continues with step 3.III. In case no additional item RI remains in the queue, which is symbolized in Figure 3 with "n", the process continues with step 3.IX, i.e. the scan is completed and the reconstructed image data MR-ID are transmitted to the medical personal. If the process continues with step 3.VII, a classification of the detected anomalies MR-IA is performed, for example using a fully connected neural network. In step 3.VIII it is determined, if the predicted probabilities $P(i)$ of the classes i of the classified anomaly data MR-CIA are all limited by a tunable probability threshold value $\theta_c$. If this is the case, which is symbolized in Figure 3 with "j", the process continues with step 3.IX. In case at least one of the probabilities $P(i)$ of the classes i exceeds the tunable probability threshold value $\theta_c$, which is symbolized in Figure 3 with "n", the process continues with step 3.X, wherein it is checked if any scan abortion flags were raised requiring to complete the scan workflow. The abortion flag can be raised based on an abortion criterion ACR, which can be a time limit that is reached, a maximum number of scan protocols reached or no scan protocol is left in the scan queue. If no abortion criterion ACR is satisfied, which is symbolized with "n" in Figure 3, the process continues with step 3.I, wherein the detected anomalies MR-CIA are taken into account for the scan recommendation of the next MR scan. In case at least one of the abortion criteria ACR is satisfied, which is symbolized with "j" in Figure 3, the process ends with step 3.IX.

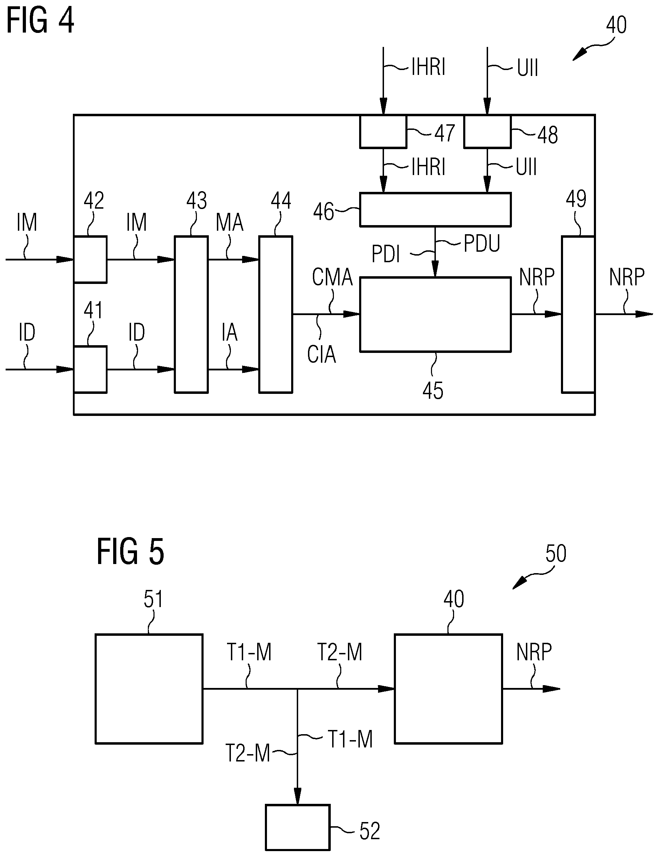

In Figure 4, a block diagram is shown, which illustrates a personal scan preparation system 40 according to an embodiment of the invention. The various blocks or modules as shown and described with reference to Figure 4 may be implemented as any suitable number of hardware circuitry, processors, and/or software (e.g. executed algorithms). The personal scan preparation system 40 comprises an image data input interface 41, which receives the above-mentioned image data ID, for example coming from an initial scan. Further, the scan recommendation system 40 comprises a metrics data input interface 42, which receives image metrics IM. Furthermore, the personal scan preparation system 40 comprises an anomaly detection unit 43, which is configured to detect some anomalies IA in the image data ID and some anomalies MA in the image metrics IM. The image anomalies IA and metrics anomalies MA are transmitted to an anomaly classification unit 44, which is configured to classify the detected anomalies IA, MA and which transmits the detected classification CIA, CMA to a scan recommendation unit 45.

In one variant, the anomaly detection unit 43 may perform the anomaly detection using as a convolutional neural network configured to process the initial images ID as an input, and to provide as output the probability of the input images ID to be normal (i.e., healthy) vs. anomalous (i.e., requiring further imaging). If the probability of anomaly IA for the provided input image ID is higher than a certain, customizable threshold value $\xi d$, the initial input image ID with marked anomalies IA may then be inserted into the subsequent anomaly classification unit 44, which classifies the anomalies IA into categories i with probability values $P(i)$ for different classes that serve a meaningful input to the scan recommendation unit 45. Suitable classification categories i may include, for instance, one or several of the following exemplary class groups: disease classes (tumor, hemorrhage, MS, etc.), anomalous anatomical regions (white matter, dark matter, brain stem, CSF, etc.), anomalous metrics (white matter volume, vascular wall thickness, carotid cross section, etc.). The classes i with high predicted classification probability $P(i)$ are then forwarded as one of the inputs to the scan recommendation unit 45. A selection of which classes are being forwarded to the scan recommendation unit 45 may be performed, e.g., by selecting a custom threshold probability value $\xi c$, such that all classes i are forwarded the probability $P(i)$ of which satisfy $P(i) > \xi c$. In another variant, the forwarding criterion for a class i may alternatively be set to satisfy

$$\max_i P(i) \text{ and } P(i) > \xi c.$$

Additionally, the personal scan preparation system 40 also comprises a third input interface 47 for receiving electronic health record information IHRI. These electronic health record information IHRI of a patient can include for example known diseases, symptoms, clinical indications for the scan, contraindications, like contrast intolerances and can be pulled for example from the RIS or another electronic system containing patient data. Furthermore, the personal scan preparation system 40 includes a manual input interface 48 as a fourth input interface for receiving some user interface input UII, for example containing suspected disease or manually selected protocols to be included in the scan workflow. The additionally received data IHRI, UII are transmitted to a keyword extraction unit 46, which extracts some relevant keywords EK-IHRI, EK-UII from the received data IHRI, UII. Further, the keywords are assigned to topics, which are mapped to possible scan workflow directions PDI based on the electronic health record information IHRI and possible scan workflow directions PDU based on the user interface input UII as evaluation values. These evaluation values are then also transmitted to the scan recommendation system 45. The scan recommendation system 45 performs an automatic scan recommendation based on a combination of various inputs CIA, CMA, PDI, PDU to identify a next recommended protocol NRP. The identified next recommended protocol NRP is output by an output interface 49, which is also part of the personal scan preparation system 40. In various embodiments, the personal scan preparation system 40 may be implemented as any suitable number of hardware circuitry, processors, and/or software (e.g. executed algorithms). As an example, the scan recommendation system 40 may be implemented as any suitable type of classical or machine learning system, such as a fully connected neural network (FCNN). In an embodiment, the personal scan preparation system 40 may work in conjunction with other classical or machine learning processes, processors, hardware components and/or interfaces, and/or additional executed algorithms to receive data via any suitable number of inputs. For instance, the personal scan preparation system 40 comprises four input interfaces 41, 42, 43, 44 and works based on four inputs ID, IM, IHRI, UII. Although this is by way of example and not limitation. Embodiments include the personal scan preparation system 40 adapted to any suitable number of inputs that may be greater than or less than four inputs and may include alternate inputs than the examples shown in Figure 4. In various embodiments, each of the input interfaces of the scan recommendation system 45 may be implemented using any suitable number of hardware circuitry, processors, and/or software (e.g. executed algorithms). In various embodiments, each of the input interfaces of the scan recommendation system 45 may be implemented as a system (e.g. a classical or machine learning system) that is best or preferably configured in accordance with the particular type of data that is received at each respective input interface.

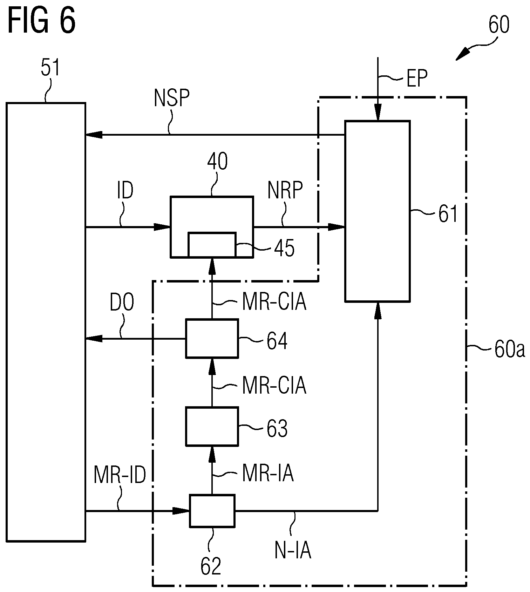

The scan recommendation system 45 may be implemented based upon, e.g., an expert system, a machine learning or deep learning approach, or combinations thereof. The scan recommendation algorithm of the scan recommendation system 45 could be, e.g., realized by one or several classical or machine learning algorithms that are able to accept the evaluation data (which were predicted by the anomaly classification units and keyword extraction units upstream) and then predict the most appropriate scan protocol to be added to a scan queue (as can be seen in Figure 6). As an example, an approach based on natural language processing with suitable word embeddings could be appropriate for this task. In an embodiment, the scan recommendation system 45 may be designed/trained, e.g., based on clinical expert knowledge and medical society guidelines.

In Figure 5, a medical imaging system 50 is schematically illustrated, comprising the personal scan preparation system 40, explained in detail in context with Figure 4. The medical imaging system 50 also comprises an MR scan system 51, which provides the personal scan preparation system 40 with T1-map data T1-M and T2-map data T2-M. Further, the medical imaging system 50 includes a contrast image generation unit 52, which generates based on the T1-map data T1-M and T2-map data T2-M, some synthetic contrast images for the use of a radiologist who is used to a certain type of image with a certain contrast. The scan recommendation system of the personal scan preparation system 40 proposes a next recommend protocol NRP for an MR imaging process.

In Figure 6 a block diagram is depicted, showing a scan workflow performing system 60 with a personal scan preparation system 40 according to an embodiment of the invention and an additional iterative protocol determination and measurement system 60a that may run until a relevant scan abortion criterion ACR combination is met. The iterative protocol determination and measurement system 60a comprises a scan queue managing unit 61, which receives a next recommended protocol NRP from the scan recommendation system 45 of the personal scan preparation system 40 and which further receives some manually or electronically selected protocols EP to be scanned and creates a scan queue, which comprises the received protocols NRP, EP. In addition to the data-driven new recommended protocol NRP, embodiments include adding protocols to the scan queue manually or electronically. This option might be required to make the scan workflow compliant, e.g., with local hospital standards, health insurance provider requirements, medical society guidelines, user preferences, etc. Hence, scan protocols NRP, EP from both channels, i.e. the personal scan preparation system 40 and the manual/electronic input, are fed into a scan queue managing unit 61. The scan queue managing unit 61 then orders and/or curates the scan protocol jobs NRP, EP according to, e.g., scheduling time, relevance, redundancies, or the input channel. The scan queue managing unit 61 may (dependent on operator and user preferences) accept both protocol input channels or, in two extreme cases, reject one or the other input channels to enforce manual or automatic mode on a high-level. Dependent on operator and user preferences, the scan queue managing unit 61 may be configured to add only certain protocols, protocols with certain parameters, or protocols satisfying specific requirements, to the scan queue. The protocols in the scan queue may be executed during the next MR scanner measurement, resulting in a reconstructed MR image. A quality assurance step and an optional rescan (if indicated) may be inserted at this point or before image reconstruction, when applicable.

Therefore, the iterative protocol determination and measurement system 60a further comprises an anomaly detection unit 62, which is configured to detect anomalies MR-IA in the MR-image data MR-ID received from an MR scan system 51. The anomaly data MR-IA are transmitted to an anomaly classification unit 63, which is also part of the iterative protocol determination and measurement system 60a. Hence, the readily reconstructed image MR-ID is inserted into the anomaly detection unit 62 (e.g., similar in function as the anomaly detection unit 43 above) suited for the respective MR scan protocol. If an anomaly MR-IA is detected, then the anomaly MR-IA may be classified by the above-mentioned subsequent anomaly classification unit 63, e.g., similar in function as the anomaly classification unit 44 above. The anomaly classification unit 63 arranges the anomaly data MR-IA in anomaly classes MR-CIA. The also called classification data MR-CIA are transmitted to an iteration unit 64, which determines based on the classification data MR-CIA and on a predetermined criterion ACR, whether the scan is completed or an additional MR scan has to be planned based on the classified anomalies MR-CIA. In case the probability $P(i)$ of the anomaly classes i of the classification data MR-CIA is smaller than a threshold value $\theta_c$, the scan is completed. If the probability $P(i)$ of at least one of the anomaly classes i is higher than the threshold $\theta_c$, it is determined, whether an abortion criterion ACR, which can be a time limit that is reached, a maximum number of scan protocols reached or no scan protocol is left in queue, is satisfied. In case one of these abortion criteria ACR is satisfied, the scan is completed. In case the abortion criteria ACR are all not satisfied, a new recommended protocol NRP is created based on the detected anomalies MR-CA. I.e. the classification data MR-CIA can be used as evaluation data for the scan recommendation system 45 of the scan preparation system 40 and a further MR scan is carried out using a newly recommended scan protocol.

Hence, following the anomaly classification, a step might be recommended that checks if any scan abortion flags were raised requiring a completion of the scan workflow, e.g., due to a time limit being reached, a maximum number of scan protocols being reached, no scan protocols being left in the queue, etc. If no scan workflow abortion criterion ACR is positive (i.e. identified), then the scan recommendation system 45 can be queried again with the classification data MR-CIA of the new scan image data MR-ID for one or several additional scan recommendation loops, until one or a relevant combination of the following criteria ACR are met and lead to a scan workflow completion:

1. No additional anomaly MR-IA is detected.

2. The classification probabilities $P(i)$ of all anomaly classes i fall below a certain threshold

$$P(i) \le \theta_c.$$

3. No item is left in the scan queue.

4. Any or a relevant combination of the scan workflow abortion criterion ACR is positive.

In case the iteration unit 64 decides that the scan has to be completed, then an order DO is transmitted to the MR scan unit 51 for transmitting the image data MR-ID to the medical personal.

In case, the anomaly detection unit 62 does not detect an anomaly MR-IA in the MR image data MR-ID from the MR scan system 51, an information N-IA is sent to the scan queue managing unit 61, which determines, if there is an additional item remaining in the queue. Then the scan queue status may decide, if another pending scan will be executed next or if the scan workflow is completed. In the first case, the scan queue managing unit 61 continues with the next protocol in the queue.

Although the repeatedly-appearing configuration of an anomaly detection unit with an anomaly classification unit connected in series is described herein, this is not the only practical configuration, and is provided by way of example and not limitation. The embodiments described herein may alternatively feed an image, image metrics, or other suitable data into the anomaly detection unit and the anomaly classification unit simultaneously for later combination of the results of both modules.

Moreover, the embodiments also include the modification of the thresholds for anomaly detection and anomaly classification (e.g., $\xi_d$, $\xi_c$, $\vartheta_d$, $\vartheta_c$) to different optimal values depending, e.g., on the position of the unit respective inside the scan workflow performing system 60, the scan protocol NSP employed for image generation, as well as other factors, a particular application, etc.

The methods and systems described herein allow for a trained or an untrained technician to perform a highly personalized MRI scan workflow on each patient, and to generate an optimal set of MRI images that are required for diagnosis of that patient's condition(s) considering potential anomalies detected on the recorded image data, electronic health record information, as well as manually- or electronically-set requirements from the side of the operator.

These techniques as discussed herein may thus provide a basis for a fully autonomous and optimized MR imaging workflow that still allows for manual or electronic customization thereof as desired by the operator. The MR scanner operator may choose to run the MR scan workflow either in fully autonomous, fully manual, or in a hybrid mode.

In an autonomous mode, dependent on the operator's preferences, by virtue of setting appropriate threshold values $\xi_c$, $\vartheta_c$, and selecting relevant scan interruption criteria ACR, the MR scan workflow may be configured to, e.g., minimize the overall scan time per patient and/or to minimize the probability of missing a relevant MR protocol required for image reading and diagnosis.

In another variant, the operator may first record a standard set of MR protocols (by means of setting workflow step 3.II, 3.III in Figure 3 appropriately) and employ the scan recommendation system 45 on demand to determine and record any additional MR scans that might be additionally indicated for the current patient.

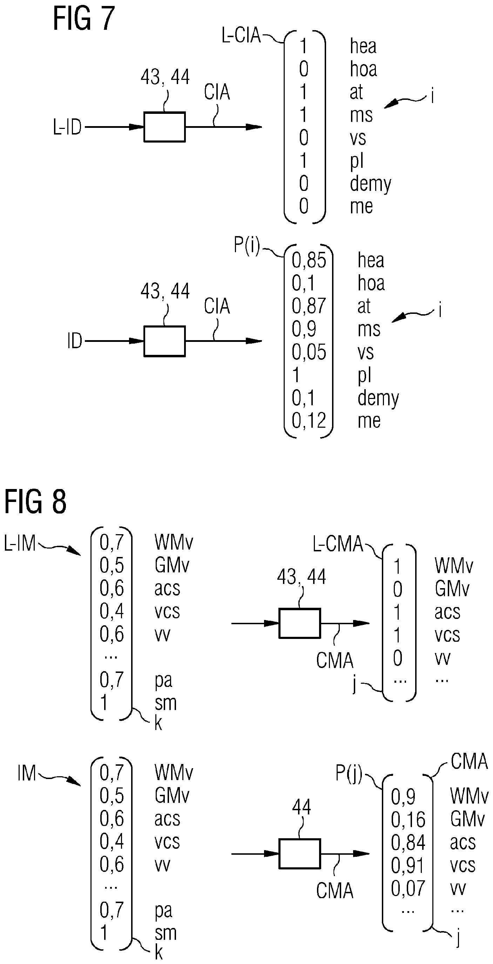

In Figure 7, an example for an image data anomaly classification is illustrated for a more detailed understanding. In the upper part of Figure 7, a training process of an image data anomaly classification is depicted. Firstly, labelled image data L-ID as training data are input to a combined anomaly detection and classification unit 43, 44. The combined anomaly detection and classification unit 43, 44 comprises a convolutional neural network, which creates classification data CIA as an answer to the labelled image data L-ID. Then the classification data CIA are compared with the labelled classification data L-CIA and the convolutional neural network is adapted such that the classification data CIA are assimilated to the labelled classification data L-CIA. In Figure 7, some classes of image abnormalities IA are depicted, for example hyperintense areas hea, hypointense areas hoa, abnormal tissues at, midline shift ms, vascular stenosis vs, plaques pl, demyelination demy and mass effects me. The anomaly classification data can be represented by a $1 \times i$-vector. Alternative, tissue specific abnormalities or anomalies IA can also be used for anomaly classification. In that case the anomalies IA are categorized by the tissue type and the anomaly type. For example, a tissue type for the brain can be white matter, grey matter, Hippocampus, Cerebellum or Corpus Callosum. The anomaly type can be hypertrophic, atrophic, or abnormal morphology. In the lower part of Figure 7, a classification process is described. Firstly, image data ID are input to the anomaly detection and classification unit 43, 44. The trained convolutional neural network of the anomaly detection and classification unit 43, 44 now outputs classification data CIA comprising a $1 \times i$-vector including probability values P(i) for the above-mentioned abnormalities IA.

In Figure 8, an example for a metrics anomaly classification is illustrated for a more detailed understanding. In the upper part of Figure 8, a training process of a metrics anomaly classification is depicted. Firstly, labelled metrics data L-IM are input to an anomaly detection and classification unit 43, 44 as training data. The anomaly detection and classification unit 43, 44 comprises a convolutional neural network, which creates classification data CMA as an answer to the labelled metrics data L-IM. Then the classification data CMA are compared with the labelled classification data L-CMA and the convolutional neural network is adapted such that the classification data CMA are assimilated to the labelled classification data L-CMA. In Figure 8, the metrics data IM comprise values concerning WMv volume, GMv volume, arterial cross section acs, venous cross section vcs, ventricular volume vv and additional information about patient age pa and sex male sm of a patient. The metrics data IM can be represented by a $1 \times k$-vector and the anomaly classification data CMA can be represented by a $1 \times j$-vector, wherein $j = k-2$, since the classification data CMA does not include information about age and sex of the patient. In the lower part of Figure 8, a classification process is described. Firstly, metrics data IM are input to the anomaly detection and classification unit 43, 44. The trained convolutional neural network of the anomaly detection and classification unit 43, 44 now outputs classification data CMA comprising a $1 \times j$-vector including probability values P(j) for the above-mentioned abnormalities MA.

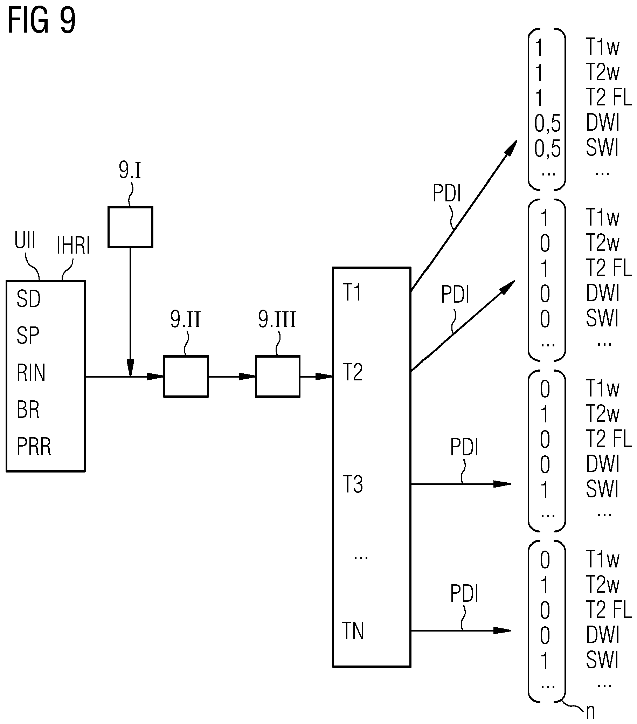

In Figure 9, an example for a keyword analysis based on electronic health record input data IHRI or user interface input data UII is illustrated in detail. The electronic health record input data IHRI can comprise information about suspected diagnosis SD, symptoms SP, referring indication RIN, the assigned body region BR and the MR protocol requested PRR. The keyword extraction starts with a data preprocessing step 9.I, for example a lemmatization and stemming. Then, in a step 9.II a bag-of-words generation is carried out. Stemming is an essential NLP (NLP = natural language programming) data preprocessing step, by which morphological variants (declensions and conjugations) of a word are traced back to the root word (stumbled -> stumble). Lemmatization is an important NLP data preparation step, reducing words from different inflectional forms to their base form (better -> good). Bag-of-words generation aims at generating an unsorted catalogue of preprocessed, cleaned words (see above) from a text document, in which the frequency of the words is resolved. After that, in step 9.III a topic detection based on a topic model is performed, wherein different topics T1, T2, T3, .., TN are detected. Then each identified topic, which is a set of keywords, is mapped to a possible scan workflow direction PDI, which is a set of recommended next scan protocols. T1w, T2w, T2 FL, DWI, SWI. This mapping can be based on expert knowledge, customer preference or an additional, designated machine learning or deep learning based algorithm. In Figure 9, the set PDI of recommended next scan protocols is represented by a $1 \times \mathbf{n}$-vector. The set PDI of recommended next scan protocols, for example comprises a T1 weighted protocol T1w, a T2 weighted protocol T2w, a T2 FLAIR protocol T2 FL, a diffusion weighted imaging protocol DWI and a susceptibility weighted imaging protocol SWI. Another possibility, if no human mapping from topics to protocols is desired or possible, is to skip topic detection 9.III and instead train a model, e.g., a convolutional neural network, that predicts protocols directly from step 9.II. This is more direct, but offers less explainability to the user.

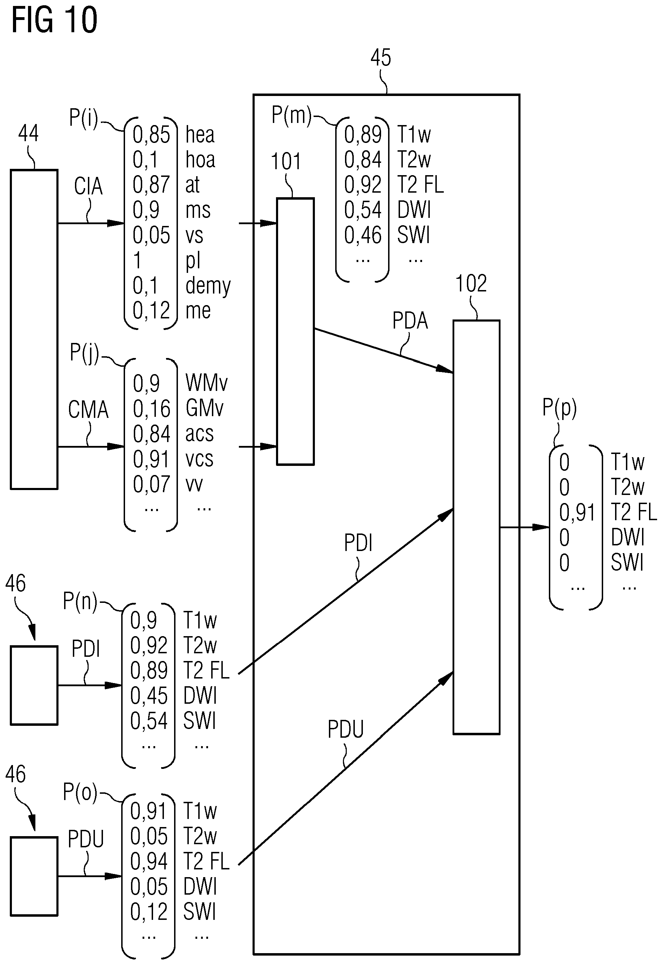

In Figure 10, the function of a scan recommendation 45 is illustrated in detail. On the left side an anomaly classification unit 44 for classification of image anomalies IA and metrics anomalies MA is depicted. Further, keyword extraction units 46 for extracting keywords from electronic health redord information data IHRI and user input data UII are depicted. As explained in context with Figure 7, 8 and 9, different classification data CIA, CMA are output by the classification unit 44 and possible scan workflow direction data PDI, PDU are output by the keyword extraction units 46. These data are input as evaluation data into the scan recommendation system 45. The anomaly classification data CIA, CMA are then processed by a mapping unit 101, which functions based on a protocol mapping model, for example a random forest model or a fully connected neural network. In this manner, some probability values $P(m)$ of next recommended protocols or possible scan workflow directions PDA are created, which have a similar format as the scan workflow direction data PDI, PDU. All these recommended next protocol data PDA, PDI, PDU are then input into a final protocol recommendation unit 102, which determines a final recommended protocol NRP by using weighted averaging or a random forest model or a fully connected neural network. As a result, a high probability value $P(p=3)$ for a T2-FLAIR protocol T2 FL is determined. Hence a T2-FLAIR protocol T2 FL is recommended for the next MR scan.

The above descriptions are merely preferred embodiments of the present disclosure but not intended to limit the present disclosure, the scope of protection of the present disclosure being solely defined by the appended claims.

**Claims**

1. Scan recommendation method, comprising the steps of:

    - receiving evaluation data comprising classification data (CIA, CMA), wherein the classification data (CIA, CMA) classify anomalies (MA) of image derived metrics (IM) detected in input data (ID, IM, IHRI, UII) concerning the medical condition of a patient, and wherein the image derived metrics include geometric properties of tissues and cavities,
    - automatic determination of a next recommended magnetic resonance protocol (NRP) to be executed in accordance with a magnetic resonance scan of the patient based on the evaluation data.

2. Scan recommendation method according to claim 1, wherein the automatic determination of a next magnetic resonance protocol (NRP) is based on using of at least one of:

    - a machine learning model, including at least one of:

        - a convolutional neural network,
        - a fully connected neural network,
        - a shallow neural network model,
        - a deep neural network model,
        - a decision tree,
        - a random forest,
        - a support vector machine,

    - an expert system,
    - a classical model algorithm.

3. The scan recommendation method according to claim 1 or 2, wherein the evaluation data comprise a first possible scan workflow direction (PDI, PDU) determined by mapping topics ($T_1$, $T_2$, $T_3$, ..., $T_N$), based on extracted keywords (EK-IHRI, EK-UII) in the input data (IHRI, UII) concerning a medical condition of the patient, onto probability values ($P(n)$, $P(o)$) for the probability of a next recommended protocol (NRP).

4. The scan recommendation method according to anyone of claim 1 to 3, wherein the automatic determination of a next recommended magnetic resonance protocol (NRP) comprises the steps of:

    - determining a second possible scan workflow direction (PDA) by automatic mapping the classification data (CIA, CMA) onto probabilities ($P(m)$) of a next recommended protocol (NRP),
    - automatic final recommending of the next recommended protocol (NRP) based on the second possible scan workflow direction (PDA) and optionally additionally based on the first possible scan workflow direction (PDI, PDU).

5. Personal scan preparation method, comprising the steps of:

    - receiving input data (ID, IM, IHRI, UII) concerning the medical condition of a patient, wherein the input data (ID, IM, IHRI, UII) comprise image-derived metrics (IM) based on image data (ID),
    - determining evaluation data based on the input data (ID, IM, IHRI, UII), comprising the steps of:

        - detecting anomalies (MA) in the image-derived metrics (IM) in the input data (ID, IM, IHRI, UII),
        - determining classification data (CMA) based on the detected anomalies (MA) as evaluation data,

    - performing the scan recommendation method according to anyone of claims 1 to 4 based on the evaluation data.

6. Personal scan preparation method according to claim 5, wherein

- the input data (ID, IM, IHRI, UII) comprise image data (ID) and
- the step of detecting anomalies (IA, MA) comprises detecting anomalies (IA) in the image data (ID).

7. Personal scan preparation method according to claim 5 or 6, wherein.

- the input data (ID, IM, IHRI, UII) further comprise electronic health record information (IHRI) and/or user interface input data (UII), and the step of determining evaluation data comprises the steps of:

- extracting keywords (EK-IHRI, EK-UII) from the electronic health record information (IHRI) and/or the user interface input (UII),

- determining topics (T1, T2, T3, ..., $T_N$) based on the extracted keywords (EK-IHRI, EK-UII),
- determining the first possible scan workflow direction (PDI, PDU) by mapping the topics (T1, T2, T3, ..., $T_N$) onto probability values (P(n), P(o)) for a probability of a next recommended protocol (NRP).

8. Medical imaging method, comprising the steps of:

- determining a next recommended protocol (NRP) based on the method of one of claims 1 to 7,
- transmitting the next recommended protocol (NRP) to a magnetic resonance scan system (51),
- performing a magnetic resonance scan by the magnetic resonance scan system (51) based on the received protocol (NRP), wherein magnetic resonance image data (MR-ID) are recorded from the patient.

9. Scan workflow performing method, comprising the steps of:

- performing the medical imaging method according to claim 8,
- detecting anomalies (MR-IA) of image derived metrics (IM) in the reconstructed magnetic resonance image data (MR-ID),
- determining classification data (MR-CIA)) based on the detected anomalies (MR-IA),
- iteratively performing the aforementioned three steps based on the determined classification data (MR-CIA) until an abortion criterion (ACR) is attained.

10. A scan recommendation system (45), comprising:

- an input interface (41, 42) for receiving evaluation data comprising classification data (CMA), wherein the classification data (CMA) classify anomalies (MA) of image derived metrics (IM) detected based on input data (ID, IM, IHRI, UII) concerning the medical condition of a patient, and wherein the image derived metrics include geometric properties of tissues and cavities,
- a recommendation unit (101, 102) for automatic determination of a next recommended magnetic resonance protocol (NRP) to be executed in accordance with an magnetic resonance scan of the patient based on the received evaluation data.

11. The scan recommendation system according to claim 10, wherein the recommendation unit (101, 102) comprises at least one of:

- a machine learning model, including at least one of:

- a convolutional neural network (CNN),
- a fully connected neural network (FCNN),
- a shallow neural network model,
- a deep neural network model,
- a decision tree,
- a random forest,
- a support vector machine,

- an expert system,
- a classical algorithm.

12. The scan recommendation system according to claim 10 or 11, comprising an input interface for receiving evaluation data comprising a first possible scan workflow direction (PDI, PDU) determined by mapping topics ($T_1$, $T_2$, $T_3$, ..., $T_N$), based on extracted keywords (EK-IHRI, EK-UII) concerning a medical condition of the patient, onto probability values (P(n), P(o)) for a probability of a next recommended protocol (NRP) .

13. The scan recommendation system according to anyone of claims 10 to 12, wherein the recommendation unit (101, 102) comprises:

- a mapping unit (101) for determining a second possible scan workflow direction (PDA) by automatic mapping of the classification data (CIA, CMA) onto probability values (P(m)) for a probability of a next recommended protocol (NRP),
- a final protocol recommendation unit (102) for final recommending of the next recommended protocol (NRP) based on the second possible scan workflow direction (PDA) and optionally additionally based on the first possible scan workflow direction (PDI, PDU).

14. Personal scan preparation system (40), comprising

- an input interface (41, 42, 47, 48) for receiving input data (ID, IM, IHRI, UII) concerning the medical condition of a patient, wherein the input data (ID, IM, IHRI, UII) comprise image-derived metrics (IM) based on image data (ID) and wherein the image derived metrics include geometric properties of tissues and cavities,
- an evaluation unit, comprising:

   - a detection unit (43) for detecting anomalies (MA) in the image derived metrics (IM) in the input data (ID, IM, IHRI, UII),
   - a classification unit (44) for determining classification data (CMA) based on the detected anomalies (MA) as evaluation data,

- the scan recommendation system (45) according to anyone of claims 9 to 13.

15. Personal scan preparation system according to claim 14,
wherein

   - the input interface (41) is configured to receive input data (ID) comprising the medical image data (ID), and
   - the detection unit (43) is configured to detect anomalies (IA) in the image data (ID).

16. Personal scan preparation system according to claim 14 or 15, wherein

   - the input interface (47, 48) is configured to receive input data (IHRI, UII) comprising electronic health record information (IHRI) and/or user interface input data (UII),
   - the evaluation unit comprises:

      - an extraction unit for extracting keywords (EK-IHRI, EK-UII) from the electronic health record information (IHRI) and/or the user interface input (UII),
      - a topic finding unit for determining topics $(T_1, T_2, T_3, ..., T_N)$ based on the extracted keywords (EK-IHRI, EK-UII),
      - a mapping unit for determining the first possible scan workflow direction by mapping the topics $(T_1, T_2, T_3, ..., T_N)$ onto probability values $(P(m), P(o))$ for a probability of a next recommended protocol (NRP) as evaluation data.

17. Medical imaging system (50), comprising:

   - a magnetic resonance scan system (51),
   - a system (40) according to anyone of claims 10 to claim 16, which is coupled to the magnetic resonance scan system (51) to determine a next recommended protocol (NRP) and to send the next recommended protocol (NRP) to the magnetic resonance scan system (51) for performing a magnetic resonance scan, wherein magnetic resonance image data (MR-ID) are recorded from a patient.

18. Scan workflow performing system (60), comprising:

   - a medical imaging system (50) according to claim 17,
   - an anomaly detection unit (62) for detecting anomalies (MR-IA) of image derived metrics (IM) in the reconstructed magnetic resonance image data (MR-ID),
   - a classification unit (63) for determining classification data (MR-CIA) based on the detected anomalies (MR-IA),
   - an iteration unit (64) for determining, if iteratively performing the three steps of medical imaging, anomaly detection and anomaly classification, based on the determined classification data (MR-CIA) until an abortion criterion (ACR) is attained.

19. Computer program product with a computer program, which can be loaded directly into a memory device of a control device of a medical imaging system (50), with program sections to carry out all steps of the method according to anyone of claims 1 to 9, when the computer program is executed in the control device.

20. Computer-readable medium, on which program sections that can be read in and executed by a computer unit of a medical imaging system (50) are stored in order to carry out all steps of the method according to anyone of claims 1 to 9, when the program sections are executed by the computer unit.

**Patentansprüche**

1. Scan-Empfehlungsverfahren, umfassend die Schritte:

   - Empfangen von Evaluierungsdaten, umfassend Klassifizierungsdaten (CIA, CMA), wobei die Klassifizierungsdaten (CIA, CMA) Anomalien (MA) von bildabgeleiteten Metriken (IM) klassifizieren, die in Eingabedaten (ID, IM, IHRI, UII) detektiert werden, die den medizinischen Zustand eines Patienten betreffen, und wobei die bildabgeleiteten Metriken geometrische Eigenschaften von Geweben und Hohlräumen einschließen,

- - automatisches Bestimmen eines nächsten empfohlenen Magnetresonanzprotokolls (NRP), das auszuführen ist, gemäß einem Magnetresonanz-Scan des Patienten basierend auf den Evaluierungsdaten.

2. Scan-Empfehlungsverfahren nach Anspruch 1, wobei das automatische Bestimmen eines nächsten Magnetresonanzprotokolls (NRP) auf der Verwendung von mindestens einem der folgenden basiert:

   - einem maschinellen Lernmodell, das mindestens eines der folgenden einschließt:

     ▪ ein neuronales Faltungsnetz,
     ▪ ein vollständig verbundenes neuronales Netz,
     ▪ ein flaches neuronales Netzmodell,
     ▪ ein tiefes neuronales Netzmodell,
     ▪ einen Entscheidungsbaum,
     ▪ einen Random Forest,
     ▪ eine Support-Vektor-Maschine,

   - ein Expertensystem,
   - einen klassischen Modellalgorithmus.

3. Scan-Empfehlungsverfahren nach Anspruch 1 oder 2, wobei die Evaluierungsdaten eine erste mögliche Scan-Arbeitsablaufrichtung (PDI, PDU) umfassen, die durch Mapping von Themen ($T_1$, $T_2$, $T_3$, ..., $T_N$) basierend auf extrahierten Schlüsselworten (EK-IHRI, EK-UII) in den Eingabedaten (IHRI, UII), die einen medizinischen Zustand des Patienten betreffen, auf Wahrscheinlichkeitswerte (P(n), P(o)) für die Wahrscheinlichkeit eines nächsten empfohlenen Protokolls (NRP) bestimmt wird.

4. Scan-Empfehlungsverfahren nach einem der Ansprüche 1 bis 3, wobei das automatische Bestimmen eines nächsten empfohlenen Magnetresonanzprotokolls (NRP) die Schritte umfasst:

   - Bestimmen einer zweiten möglichen Scan-Arbeitsablaufrichtung (PDA) durch automatisches Mapping der Klassifizierungsdaten (CIA, CMA) auf Wahrscheinlichkeiten (P(m)) eines nächsten empfohlenen Protokolls (NRP),
   - automatisches endgültiges Empfehlen des nächsten empfohlenen Protokolls (NRP) basierend auf der zweiten möglichen Scan-Arbeitsablaufrichtung (PDA) und gegebenenfalls zusätzlich basierend auf der ersten möglichen Scan-Arbeitsablaufrichtung (PDI, PDU).

5. Persönliches Scan-Vorbereitungsverfahren, umfassend die Schritte:

   - Empfangen von Eingabedaten (ID, IM, IHRI, UII), die den medizinischen Zustand eines Patienten betreffen, wobei die Eingabedaten (ID, IM, IHRI, UII) bildabgeleitete Metriken (IM) umfassen, die auf Bilddaten (ID) basieren,
   - Bestimmen von Evaluierungsdaten basierend auf den Eingabedaten (ID, IM, IHRI, UII), umfassend die Schritte:

     • Detektieren von Anomalien (MA) in den bildabgeleiteten Metriken (IM) in den Eingabedaten (ID, IM, IHRI, UII),
     • Bestimmen von Klassifizierungsdaten (CMA) basierend auf den detektierten Anomalien (MA) als Evaluierungsdaten,

   - Durchführen des Scan-Empfehlungsverfahrens gemäß einem der Ansprüche 1 bis 4 basierend auf den Evaluierungsdaten.

6. Persönliches Scan-Vorbereitungsverfahren nach Anspruch 5, wobei

   - die Eingabedaten (ID, IM, IHRI, UII) Bilddaten (ID) umfassen, und
   - der Schritt des Detektierens von Anomalien (IA, MA) Detektieren von Anomalien (IA) in den Bilddaten (ID) umfasst.

7. Persönliches Scan-Vorbereitungsverfahren nach Anspruch 5 oder 6, wobei die Eingabedaten (ID, IM, IHRI, UII) des Weiteren Informationen aus der elektronischen Gesundheitsakte (IHRI) und/oder Benutzerschnittstelleneingabedaten (UII) umfassen, und der Schritt des Bestimmens von Evaluierungsdaten die Schritte umfasst:

   - Extrahieren von Schlüsselworten (EK-IHRI, EK-UII) aus Informationen aus der elektronischen Gesundheitsakte (IHRI) und/oder der Benutzerschnittstelleneingabe (UII),
   - Bestimmen von Themen (T1, T2, T3, ..., $T_N$) basierend auf den extrahierten Schlüsselworten (EK-IHRI, EK-UII),
   - Bestimmen der ersten möglichen Scan-Arbeitsablaufrichtung (PDI, PDU) durch Mapping der Themen (T1, T2, T3, ..., $T_N$) auf Wahrscheinlichkeitswerte (P(n), P(o)) für eine Wahrscheinlichkeit eines nächsten empfohlenen Protokolls (NRP).

8. Medizinisches Bildgebungsverfahren, umfassend die Schritte:

   - Bestimmen eines nächsten empfohlenen Protokolls (NRP) basierend auf dem Verfahren gemäß einem der Ansprüche 1 bis 7,
- Übermitteln des nächsten empfohlenen Protokolls (NRP) an ein Magnetresonanz-Scansystem (51),
- Durchführen eines Magnetresonanz-Scans durch das Magnetresonanz-Scansystem (51) basierend auf dem empfangenen Protokoll (NRP), wobei Magnetresonanzbilddaten (MR-ID) von dem Patienten aufgezeichnet werden.

9. Scan-Arbeitsablaufdurchführungsverfahren, umfassend die Schritte:

   - Durchführen des medizinischen Bildgebungsverfahrens nach Anspruch 8,
   - Detektieren von Anomalien (MR-IA) von bildabgeleiteten Metriken (IM) in den rekonstruierten Magnetresonanzbilddaten (MR-ID),
   - Bestimmen von Klassifizierungsdaten (MR-CIA) basierend auf den detektierten Anomalien (MR-IA),
   - iteratives Durchführen der genannten drei Schritte basierend auf den bestimmten Klassifizierungsdaten (MR-CIA), bis ein Abbruchkriterium (ACR) erreicht ist.

10. Scan-Empfehlungssystem (45), umfassend:

    - eine Eingabeschnittstelle (41, 42) zum Empfangen von Evaluierungsdaten, umfassend Klassifizierungsdaten (CMA), wobei die Klassifizierungsdaten (CMA) Anomalien (MA) von bildabgeleiteten Metriken (IM) klassifizieren, die basierend auf Eingabedaten (ID, IM, IHRI, UII) detektiert werden, die den medizinischen Zustand eines Patienten betreffen, und wobei die bildabgeleiteten Metriken geometrische Eigenschaften von Geweben und Hohlräumen einschließen,
    - eine Empfehlungseinheit (101, 102) zum automatischen Bestimmen eines nächsten empfohlenen Magnetresonanzprotokolls (NRP), das auszuführen ist, gemäß einem Magnetresonanz-Scan des Patienten basierend auf den empfangenen Evaluierungsdaten.

11. Scan-Empfehlungssystem nach Anspruch 10, wobei die Empfehlungseinheit (101, 102) mindestens eines der folgenden umfasst:

    - ein maschinelles Lernmodell, das mindestens eines der folgenden einschließt:

      • ein neuronales Faltungsnetz (CNN),
      • ein vollständig verbundenes neuronales Netz (FCNN),
      • ein flaches neuronales Netzmodell,

      • ein tiefes neuronales Netzmodell,
      • einen Entscheidungsbaum,
      • einen Random Forest,
      • eine Support-Vektor-Maschine,

    - ein Expertensystem,
    - einen klassischen Algorithmus.

12. Scan-Empfehlungssystem nach Anspruch 10 oder 11, umfassend eine Eingabeschnittstelle zum Empfangen von Evaluierungsdaten, umfassend eine erste mögliche Scan-Arbeitsablaufrichtung (PDI, PDU), die durch Mapping von Themen ($T_1$, $T_2$, $T_3$, ..., $T_N$) basierend auf extrahierten Schlüsselworten (EK-IHRI, EK-UII), die einen medizinischen Zustand des Patienten betreffen, auf Wahrscheinlichkeitswerte (P(n), P(o)) für die Wahrscheinlichkeit eines nächsten empfohlenen Protokolls (NRP) bestimmt wird.

13. Scan-Empfehlungssystem nach einem der Ansprüche 10 bis 12, wobei die Empfehlungseinheit (101, 102) umfasst:

    - eine Mapping-Einheit (101) zum Bestimmen einer zweiten möglichen Scan-Arbeitsablaufrichtung (PDA) durch automatisches Mapping der Klassifizierungsdaten (CIA, CMA) auf Wahrscheinlichkeitswerte (P(m)) für eine Wahrscheinlichkeit eines nächsten empfohlenen Protokolls (NRP),
    - eine endgültige Protokollempfehlungseinheit (102) zur endgültigen Empfehlung des nächsten empfohlenen Protokolls (NRP) basierend auf der zweiten möglichen Scan-Arbeitsablaufrichtung (PDA) und gegebenenfalls zusätzlich basierend auf der ersten möglichen Scan-Arbeitsablaufrichtung (PDI, PDU).

14. Persönliches Scan-Vorbereitungssystem (40), umfassend:

    - eine Eingabeschnittstelle (41, 42, 47, 48) zum Empfangen von Eingabedaten (ID, IM, IHRI, UII), die den medizinischen Zustand eines Patienten betreffen, wobei die Eingabedaten (ID, IM, IHRI, UII) bildabgeleitete Metriken (IM) umfassen, die auf Bilddaten (ID) basieren, und wobei die bildabgeleiteten Metriken geometrische Eigenschaften von Geweben und Hohlräumen einschließen,
    - eine Evaluierungseinheit, umfassend:

      • eine Detektierungseinheit (43) zum Detektieren von Anomalien (MA) in den bildabgeleiteten Metriken (IM) in den Eingabedaten (ID, IM, IHRI, UII),
      • eine Klassifizierungseinheit (44) zum Bestimmen von Klassifizierungsdaten (CMA) basierend auf den detektierten Anomalien (MA) als Evaluierungsdaten,

- das Scan-Empfehlungssystem (45) gemäß einem der Ansprüche 9 bis 13.

15. Persönliches Scan-Vorbereitungssystem nach Anspruch 14, wobei

- die Eingabeschnittstelle (41) ausgelegt ist, um Eingabedaten (ID) zu empfangen, die die medizinischen Bilddaten (ID) umfassen, und
- die Detektierungseinheit (43) ausgelegt ist, um Anomalien (IA) in den Bilddaten (ID) zu detektieren.

16. Persönliches Scan-Vorbereitungssystem nach Anspruch 14 oder 15, wobei

- die Eingabeschnittstelle (47, 48) ausgelegt ist, um Eingabedaten (IHRI, UII) zu empfangen, die Informationen aus der elektronischen Gesundheitsakte (IHRI) und/oder Benutzerschnittstelleneingabedaten (UII) umfassen,
- die Evaluierungseinheit umfasst:

• eine Extraktionseinheit zum Extrahieren von Schlüsselworten (EK-IHRI, EK-UII) aus Informationen aus der elektronischen Gesundheitsakte (IHRI) und/oder der Benutzerschnittstelleneingabe (UII),
• eine Themenfindungseinheit zum Bestimmen von Themen ($T_1$, $T_2$, $T_3$, ..., $T_N$) basierend auf den extrahierten Schlüsselworten (EK-IHRI, EK-UII),
• eine Mapping-Einheit zum Bestimmen der ersten möglichen Scan-Arbeitsablaufrichtung durch Mapping der Themen ($T_1$, $T_2$, $T_3$, ..., $T_N$) auf Wahrscheinlichkeitswerte (P(m), P(o)) für eine Wahrscheinlichkeit eines nächsten empfangenen Protokolls (NRP) als Evaluierungsdaten.

17. Medizinisches Bildgebungssystem (50), umfassend:

- ein Magnetresonanz-Scansystem (51),
- ein System (40) gemäß einem der Ansprüche 10 bis Anspruch 16, das an das Magnetresonanz-Scansystem (51) gekoppelt wird, um ein nächstes empfohlenes Protokoll (NRP) zu bestimmen und das nächste empfohlene Protokoll (NRP) an das Magnetresonanz-Scansystem (51) zu senden, um einen Magnetresonanz-Scan durchzuführen, wobei Magnetresonanzbilddaten (MR-ID) von einem Patienten aufgezeichnet werden.

18. Scan-Arbeitsablaufdurchführungssystem (60), umfassend:

- ein medizinisches Bildgebungssystem (50) gemäß Anspruch 17,
- eine Anomaliedetektierungseinheit (62) zum Detektieren von Anomalien (MR-IA) von bildabgeleiteten Metriken (IM) in den rekonstruierten Magnetresonanzbilddaten (MR-ID),
- eine Klassifizierungseinheit (63) zum Bestimmen von Klassifizierungsdaten (MR-CIA) basierend auf den detektierten Anomalien (MR-IA),
- eine Iterationseinheit (64) zum Bestimmen der Anomaliedetektierung und Anomalieklassifizierung, falls die drei Schritte der medizinischen Bildgebung iterativ durchgeführt werden, basierend auf den bestimmten Klassifizierungsdaten (MR-CIA), bis ein Abbruchkriterium (ACR) erreicht ist.

19. Computerprogrammprodukt mit einem Computerprogramm, das direkt in eine Speichervorrichtung einer Steuervorrichtung eines medizinischen Bildgebungssystems (50) geladen werden kann, mit Programmabschnitten, um alle Schritte des Verfahrens gemäß einem der Ansprüche 1 bis 9 durchzuführen, wobei das Computerprogramm in der Steuervorrichtung ausgeführt wird.

20. Computerlesbares Medium, auf dem Programmabschnitte, die von einer Computereinheit eines medizinischen Bildgebungssystems (50) eingelesen und ausgeführt werden können, gespeichert sind, um alle Schritte des Verfahrens gemäß einem der Ansprüche 1 bis 9 durchzuführen, wenn die Programmabschnitte von der Computereinheit ausgeführt werden.

**Revendications**

1. Procédé de recommandation de scan, comprenant les stades de :

- réception de données d'évaluation comprenant des données (CIA, CMA) de classification, dans lequel les données (CIA, CMA) de classification classent des anomalies (MA) de métriques (IM) déduites d'une image détectées dans des données (ID, IM, IHRI, UII) d'entrée concernant l'état médical d'un patient, et dans lequel les métriques déduites d'images comprennent des propriétés géométriques de tissus et de cavités,
- détermination automatique d'un protocole (NRP) de résonnance magnétique suivant recommandé à exécuter en accord avec un scan de résonnance magnétique du patient sur la base des données d'évaluation.

**2.** Procédé de recommandation de scan suivant la revendication 1, dans lequel la détermination automatique d'un protocole (NRP) de résonnance magnétique suivant repose sur l'utilisation d'au moins l'un de :

- un modèle d'apprentissage automatique, comprenant au moins l'un de :

- un réseau neuronal convolutif,
- un réseau neuronal entièrement connecté,
- un modèle de réseau neuronal superficiel,
- un modèle de réseau neuronal profond,
- un arbre de décision,
- une forêt d'arbres décisionnels,
- une machine à vecteurs de support,

- un système expert,
- un algorithme de modèle classique.

**3.** Procédé de recommandation de scan suivant la revendication 1 ou 2, dans lequel les données d'évaluation comprennent un premier sens (PDI, PDU) possible de plan de circulation de scan déterminé par des sujets ($T_1$, $T_2$, $T_3$, ..., $T_N$) de topographie, reposant sur des mots-clés (EK-IHRI, EK-UII) extraits dans la donnée (IHRI, UII) d'entrée concernant un état médical du patient, sur des valeurs (P(n), P(o)) de probabilité pour la probabilité d'un protocole (NRP) suivant recommandé.

**4.** Procédé de recommandation de scan suivant l'une quelconque des revendications 1 à 3, dans lequel la détermination automatique d'un protocole (NRP) suivant de résonnance magnétique recommandé comprend les stades de :

- détermination d'un deuxième sens (PDA) de plan de circulation de scan par topographie automatique des données (CIA, CMA) de classification sur des probabilités (P(m)) d'un protocole (NRP) suivant recommandé,
- recommandation finale automatique du protocole (NRP) suivant recommandé sur la base du deuxième sens possible (PDA) de plan de circulation de scan et sur la base en outre éventuellement du premier sens (PDI, PDU) possible de plan de circulation de scan.

**5.** Procédé de préparation d'un scan personnel, comprenant les stades de :

- réception des données (ID, IM, IHRI, UII) d'entrée concernant l'état médical d'un patient, dans lequel les données (ID, IM, IHRI, UII) d'entrée comprennent des métriques (IM) déduites d'une image sur la base des données (ID) d'image,
- détermination de données d'évaluation sur la base des données (ID, IM, IHRI, UII) d'entrée comprenant les stades de :

- détection d'anomalies (MA) dans les métriques (IM) déduites d'une image dans les données (ID, IM, IHRI, UII) d'entrée,
- détermination de données (CMA) de classification sur la base des anomalies (MA) détectées comme données d'évaluation,

- effectuer le procédé de recommandation de scan suivant l'une quelconque des revendications 1 à 4 sur la base des données d'évaluation.

**6.** Procédé de préparation d'un scan personnel suivant la revendication 5, dans lequel

- les données (ID, IM, IHRI, UII) d'entrée comprennent des données (ID) d'image, et
- le stade de détection d'anomalies (IA, MA) comprend détecter des anomalies (IA) dans les données (ID) d'image.

**7.** Procédé de préparation d'un scan personnel suivant la revendication 5 ou 6, dans lequel

- les données (ID, IM, IHRI, UII) d'entrée comprennent en outre une information (IHRI) électronique d'enregistrement de santé et/ou des données d'entrée (UII) d'interface d'utilisateur , et le stade de détermination des données d'évaluation comprend les stades de :
- extraction des mots-clés (EK-IHRI, EK-UII) de l'information (IHRI) électronique d'enregistrement de santé et/ou de l'entrée (UII) d'interface d'utilisateur,
- déterminer des sujets (T1, T2, T3, ... $T_N$) sur la base des mots-clés (EK-IHRI, EK-UII) extraits,
- déterminer le premier sens (PDI, PDU) possible de plan de circulation de scan en topographiant les sujets (T1, T2, T3, ..., $T_N$) sur des valeurs (P (n), P (o)) de probabilité pour une probabilité d'un protocole (NRP) suivant recommandé.

**8.** Procédé d'imagerie médicale, comprenant les stades :

- déterminer un protocole (NRP) recommandé suivant sur la base du procédé suivant l'une des revendications 1 à 7,
- transmettre du protocole (NRP) recommandé suivant à un système (51) de scan à résonnance magnétique,
- effectuer un scan à résonnance magnétique par le système (51) de scan à résonnance magnétique sur la base du protocole (NRP) reçu, dans lequel des données (MR-ID) d'image de résonnance magnétique sont enregistrées du patient.

9. Procédé pour effectuer un plan de circulation de scan, comprenant les stades :

- effectuer le procédé d'imagerie médicale suivant la revendication 8,
- détecter des anomalies (MR-IA) de métriques (IM) déduites d'une image dans les données (MR-ID) de résonnance magnétique reconstruites,
- déterminer des données (MR-CIA) de classification sur la base des anomalies (MR-IA) détectées,
- effectuer itérativement les trois stades mentionnés ci-dessus, sur la base des données (MR-CIA) de classification déterminées jusqu'à atteindre un critère (ACR) de fin.

10. Système (45) de recommandation de scan, comprenant :

- une interface (41, 42) d'entrée pour recevoir des données d'évaluation comprenant des données (CMA) de classification, dans lequel les données (CMA) de classification classe des anomalies (MA) de métriques (IM) déduites d'une image détectées sur la base des données (ID, IM, IHRI, UII) d'entrée concernant l'état médical d'un patient, et dans lequel les métriques déduites d'une image comprennent des propriétés géométriques de tissus et de cavités,
- une unité (101, 102) de recommandation pour la détermination automatique d'un protocole (NRP) suivant de résonnance magnétique recommandé à exécuter en accord avec un scan de résonnance magnétique du patient sur la base des données d'évaluation reçues.

11. Système de recommandation de scan suivant la revendication 10,
dans lequel l'unité (101, 102) comprend au moins l'un de :

- un modèle d'apprentissage automatique, comprenant au moins l'un de :

- un réseau neuronal convolutif (CNN),
- un réseau neuronal entièrement connecté (FCNN),
- un modèle de réseau neuronal superficiel,
- un modèle de réseau neuronal profond,
- un arbre de décision,
- une forêt d'arbres décisionnels,

- une machine à vecteurs de support,

- un système expert,
- un algorithme de modèle classique.

12. Système de recommandation de scan suivant la revendication 10 ou 11, comprenant une interface d'entrée pour recevoir des données d'évaluation comprenant un premier sens (PDI, PDU) possible de plan de circulation de scan déterminé par des objets ($T_1$, $T_2$, $T_3$, ..., $T_N$) de topographie, reposant sur des mots-clés (EK-IHRI, EK-UII) extraits concernant un état médical du patient, sur des valeurs (P(n), P(o)) de probabilité pour la probabilité d'un protocole (NRP) suivant recommandé.

13. Système de recommandation de scan suivant l'une quelconque des revendications 10 à 12,
dans lequel l'unité (101, 102) de recommandation comprend :

- une unité (101) de topographie pour déterminer un deuxième sens (PDA) de plan de circulation de scan possible par topographie automatique des données (CIA, CMA) de classification sur des valeurs (P(m)) de probabilité pour une probabilité d'un protocole (NRP) suivant recommandé,
- une unité (102) finale de recommandation de protocole pour recommander finalement le protocole (NRP) suivant recommandé sur la base du deuxième sens (PDA) possible de plan de circulation de scan et éventuellement en outre sur la base du premier sens (PDI, PDU) possible de plan de circulation de scan.

14. Système (40) de préparation de scan personnel, comprenant :

- une interface (41, 42, 47, 48) d'entrée pour recevoir des données (ID, IM, IHRI, UII) d'entrée concernant l'état médical d'un patient, dans lequel les données (ID, IM, IHRI, UII) d'entrée comprennent des métriques (IM) déduites d'une image sur la base des données (ID) d'image et dans lequel les métriques déduites d'une image comprennent des propriétés géométriques de tissus et de cavités,
- une unité d'évaluation comprenant :

- une unité (43) de détection pour détecter des anomalies (MA) dans les métriques (IM) déduites d'une image dans les données (ID, IM, IHRI, UII) d'entrée,
- une unité (44) de classification pour déterminer des données (CMA) de classification sur la base des anomalies (MA) détectées comme données d'évaluation,

- le système (45) d'évaluation de scan suivant l'une quelconque des revendications 9 à 13.

15. Système de préparation de scan personnel suivant la revendication 14, dans lequel

- l'interface (41) d'entrée est configurée pour recevoir des données (ID) d'entrée comprenant les données (ID) d'image médicale, et
- l'unité (43) de détection est configurée pour détecter des anomalies (IA) dans les données (ID) d'image médicale.

16. Système de préparation de scan personnel suivant la revendication 14 ou 15, dans lequel

- l'interface (47, 48) d'entrée est configurée pour recevoir des données (IHRI, UII) d'entrée comprenant de l'information d'enregistrement (IHRI) électronique de santé et/ou des données (UII) d'interface d'utilisateur,
- l'unité d'évaluation comprenant :

     - une unité d'extraction pour extraire des mots-clés (EK-IHRI, EK-UII) de d'information (IHRI) d'enregistrement électronique de santé et/ou de l'entrée (UII) d'interface d'utilisateur,
     - une unité pour trouver des sujets pour déterminer des sujets ($T_1$, $T_2$, $T_3$, ..., $T_N$) sur la base des mots-clés (EK-IHRI, EK-UII) extraits,
     - une unité de topographie pour déterminer le premier sens possible de plan de circulation de scan en topographiant les sujets ($T_1$, $T_2$, $T_3$, ..., $T_N$) sur des valeurs (P(m), P(o)) de probabilité pour une probabilité d'un protocole (NRP) suivant recommandé comme données d'évaluation.

17. Système (50) d'imagerie médicale, comprenant :

     - un système (51) de scan à résonance magnétique,
     - un système (40) suivant l'une quelconque des revendications 10 à la revendication 16, qui est reliée au système (51) de scan à résonnance magnétique pour déterminer un protocole (NRP) suivant recommandé et pour envoyer le protocole (NRP) suivant recommandé au système (51) de scan à résonnance magnétique pour effectuer un scan de résonnance magnétique, dans lequel les données (MR-ID) d'image de résonnance magnétique sont enregistrées chez un patient.

18. Système (60) pour effectuer un plan de circulation de scan, comprenant :

- un système (50) d'imagerie médicale suivant la revendication 17,
- une unité (62) de détection d'anomalies pour détecter des anomalies (MR-IA) de métriques (IM) déduites d'une image dans les données (MR-ID) de résonnance magnétique reconstruites,
- une unité (63) de classification pour déterminer des données (MR-CIA) de classification sur la base des anomalies (MR-IA) détectées,
- une unité (64) d'itération pour déterminer, si l'on effectue itérativement les trois stades d'imagerie médicale, une détection d'anomalies et une classification d'anomalies sur la base des données (MR-CIA) de classification déterminées jusqu'à ce qu'une critère (ACR) de fin soit atteint.

19. Produit de programme d'ordinateur ayant un programme d'ordinateur, qui peut être chargé directement dans un dispositif de mémoire d'un dispositif de commande d'un système (50) d'imagerie médicale, ayant des parties de programme pour effectuer tous les stades du procédé suivant l'une quelconque des revendications 1 à 9, lorsque le programme d'ordinateur est exécuté dans le dispositif de commande.

20. Support, déchiffrable par ordinateur, sur lequel des parties de programme, qui peuvent être entrées et exécutées par une unité informatique d'un système (50) d'imagerie médicale sont mises en mémoire afin d'effectuer tous les stades du procédé suivant l'une quelconque des revendications 1 à 9, lorsque les parties du programme sont exécutées par l'unité informatique.

FIG 1

100

1.I

ID

ID

1.II

IA

1.V

CIA

1.III

IM

1.IV

MA

1.VI

CMA

1.VII

IHRI

EK-IHRI ─ 1.IX

PDI

1.VIII

UII

EK-UII ─ 1.X

PDU

1.XI

FIG 2

100a

1.Ia

T1-M

1.Ic

T2-M-A    T1-M-A

1.Id ─ SCA

1.Ib

T2-M

T1-M-A    T2-M-A

1.II

T1-M-A    T2-M-A

1.III

# FIG 3

EP 3 901 964 B1

FIG 4

40

IHRI          UII

47      48

IHRI      UII

42        43        44        46                              49

IM      IM      MA              PDI        PDU

CMA                        NRP        NRP

41        ID        IA

ID                CIA

45

FIG 5

50

51                                    40

T1-M      T2-M              NRP

T2-M      T1-M

52

24

# FIG 6

## FIG 7

L-ID → [43, 44] → CIA →

L-CIA
$$\begin{pmatrix} 1 \\ 0 \\ 1 \\ 1 \\ 0 \\ 1 \\ 0 \\ 0 \end{pmatrix}$$
hea
hoa
at
ms ← i
vs
pl
demy
me

ID → [43, 44] → CIA →

P(i)
$$\begin{pmatrix} 0,85 \\ 0,1 \\ 0,87 \\ 0,9 \\ 0,05 \\ 1 \\ 0,1 \\ 0,12 \end{pmatrix}$$
hea
hoa
at
ms ← i
vs
pl
demy
me

## FIG 8

L-IM →
$$\begin{pmatrix} 0,7 \\ 0,5 \\ 0,6 \\ 0,4 \\ 0,6 \\ ... \\ 0,7 \\ 1 \end{pmatrix}$$
WMv
GMv
acs
vcs
vv

pa
sm
k

→ [43, 44] → CMA →

L-CMA
$$\begin{pmatrix} 1 \\ 0 \\ 1 \\ 1 \\ 0 \\ ... \end{pmatrix}$$
WMv
GMv
acs
vcs
vv
...
j

IM →
$$\begin{pmatrix} 0,7 \\ 0,5 \\ 0,6 \\ 0,4 \\ 0,6 \\ ... \\ 0,7 \\ 1 \end{pmatrix}$$
WMv
GMv
acs
vcs
vv

pa
sm
k

→ [44] → CMA →

P(j)
$$\begin{pmatrix} 0,9 \\ 0,16 \\ 0,84 \\ 0,91 \\ 0,07 \\ ... \end{pmatrix}$$
CMA
WMv
GMv
acs
vcs
vv
...
j

EP 3 901 964 B1

FIG 9

9.I

UII    IHRI

SD

SP

RIN

BR

PRR

9.II    9.III

T1

T2

T3

...

TN

PDI

PDI

PDI

PDI

| 1 | T1w |
| 1 | T2w |
| 1 | T2 FL |
| 0,5 | DWI |
| 0,5 | SWI |
| ... | ... |

| 1 | T1w |
| 0 | T2w |
| 1 | T2 FL |
| 0 | DWI |
| 0 | SWI |
| ... | ... |

| 0 | T1w |
| 1 | T2w |
| 0 | T2 FL |
| 0 | DWI |
| 1 | SWI |
| ... | ... |

| 0 | T1w |
| 1 | T2w |
| 0 | T2 FL |
| 0 | DWI |
| 1 | SWI |
| ... | ... |

n

# FIG 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63013825 B
- US 9928589 B2
- US 20180101644 A1
- US 2019320934 A1

### Non-patent literature cited in the description

- Efficiency Improvement in a Busy Radiology Practice: Determination of Musculoskeletal Magnetic Resonance Imaging Protocol Using Deep-Learning Convolutional Neural Networks. **LEE YOUNG HAN.** JOURNAL OF DIGITAL IMAGING. SPRINGER-VERLAG, 04 April 2018, vol. 31, 604-610
- **D. BLEI et al.** *JMLR,* 2003, vol. 3
- **Y. MIAO et al.** *Proc. ICML,* 2017